# EUROPÄISCHE PATENTSCHRIFT

(11) **EP 0 924 518 B1**
(45) Veröffentlichungstag und Bekanntmachung des Hinweises auf die Patenterteilung: **08.11.2006**
(21) Anmeldenummer: 98123337.2
(22) Anmeldetag: 08.12.1998
(51) Int. Cl.: G01N 33/36, G01N 27/22

(54) **Vorrichtung zum Messen von Eigenschaften eines textilen Produktes**
Apparatus for measuring characteristics of a textile product
Appareil pour la mesure de caractéristiques d'un produit textile

(30) Priorität: 19.12.1997 CH 292697
(43) Veröffentlichungstag der Anmeldung: 23.06.1999
(73) Patentinhaber: Uster Technologies AG, 8610 Uster (CH)
(72) Erfinder: Schöni, Markus, 8603 Schwerzenbach (CH)
(74) Vertreter: Ellenberger, Maurice

(56) Entgegenhaltungen:
- DE-A- 1 448 061
- DE-A- 2 541 261
- GB-A- 1 373 922
- DATABASE WPI Week 9424 Derwent Publications Ltd., London, GB; AN 94-198111 [24] XP002076593 & SU 1 805 146 A (POISK RES INST) , 19. Februar 1990

## Beschreibung

Die Erfindung betrifft eine Vorrichtung zum Messen von Eigenschaften eines textilen Produktes in einem Messspalt in dem das textile Produkt eingeführt ist.

Eine solche Vorrichtung ist aus der DE-A-36 21 324 bekannt. Dabei sind zwei Kondensatoren, ein Messkondensator und ein Kompensationskondensator, vorgesehen, die beide an je eine eigene Oszillatorschaltung angeschlossen sind und zwischen den Elektroden oder Kondensatorplatten je einen eigenen Spalt aufweisen. Das textile Produkt wird durch den Spalt des Messkondensators hindurchbewegt und dabei gemessen. Der Spalt des Messkondensators und der Spalt des Kompensationskondensators sind beide je mit einer Einrichtung versehen, mit der die Breite des Spaltes eingestellt werden kann. Diese Einrichtung ist so ausgebildet, dass sich beide Spalte im gleichen Masse öffnen oder schliessen. Damit kann die Vor richtung auf verschiedene Masse des textilen Produktes eingestellt werden.

Der Nachteil dieser bekannten Vorrichtung besteht darin, dass die genannte Einrichtung zum Verändern des Abstandes zwischen den Platten der beiden Kondensatoren sehr aufwendig und schwer ist. Diese Einrichtung muss genügend genau arbeiten um Änderungen der Spaltbreite am Messkondensator möglichst genau in eine Änderung der Spaltbreite am Kompensationskondensator umzusetzen. Mechanische Änderungen der Spaltbreite, die während der Messung evtl. ungewollt geschehen, können mit dieser Vorrichtung nicht kompensiert werden. Dies gilt ebenso für andere Änderungen der Verhältnisse in den beiden Spalten, die nicht an beiden Spalten gleichzeitig geschehen, wie z.B. Feuchtigkeitsveränderungen im Messspalt, die auf feuchtes Gam zurückzuführen sind und die nur lokal auftreten.

Aus der CH 551 007 ist ein kapazitiver Messkopf für Gam bekannt, bei dem symmetrisch und damit beidseitig eines Messkondensators je ein Kompensationskondensator vorgesehen ist. Die Elektroden sind viel breiter als das Garn ausgebildet um im Messkondensator ein möglichst homogenes Feld zu erzeugen. Um störende Felder zu vermeiden, sind die Anschlüsse oder Zuführungen zu den Elektroden des Messkondensators und des Kompensationskondensators in oder hinter das Trägermaterial, das die Elektroden festhält, verlegt.

Ein Nachteil dieser weiteren bekannten Anordnung ist deshalb darin zu sehen, dass im Messkopf und damit auch im Messspalt eine relativ grosse Fläche mit Elektroden besetzt ist und dass die Zuleitungen zu den Elektroden, die senkrecht dazu an die Elektroden zu und weggeführt sind, eine Bauweise ergeben, die insgesamt viel Raum beansprucht und schwierig zu herzustellen ist.

Die Erfindung wie sie in den Patentansprüchen gekennzeichnet ist, löst nun die Aufgabe, eine möglichst genau arbeitende und unempfindliche Vorrichtung zu schaffen, bei der die Wirkungen von Änderungen der Breite des Spaltes im Messkondensator auf einfache Art korrigiert werden und die auch eine raumsparende Anordnung der Elektroden und Zuführungen ermöglicht.

Diese Aufgabe wird dadurch gelöst, dass der Messspalt nun Elektroden eines Messkondensators, Elektroden eines Kompensationskondensators und Leitungen zu beiden Kondensatoren aufweist, die in einer Ebene mit den Elektroden angeordnet sind.

Die durch die Erfindung erreichten Vorteile sind insbesondere darin zu sehen, dass sich Messfehler, die durch Änderungen der Spaltbreite im Messkondensator oder durch Unterschiede in der Luftfeuchtigkeit, der Temperatur oder der Luftströmung entstehen können, automatisch kompensieren. Die vorgeschlagene Lösung setzt keine mechanisch bewegten Teile voraus und lässt sich an die speziellen Verhältnisse im Messspalt gut anpassen. Aufgrund ihrer Einfachheit und ihrer raumsparenden Anordnung, kann diese Lösung auch an Vorrichtungen mit mehreren Messspalten vorgesehen werden. Damit kann nun die Anpassung der Messvorrichtung auf verschiedene Masse auch dadurch geschehen, dass Messspalte verschiedener Breite fest vorgesehen werden und dass je nach Querschnitt, das textile Produkt in einen besonders dafür geeigneten Messspalt geleitet wird. Durch die vorgeschlagene Lösung ist auch automatisch dafür gesorgt, dass das Signal, das bei der Messung entsteht, als Folge von Störeinflüssen nicht abdriftet. Durch geeignete Anordnung der verschiedenen Elektroden, die die Kondensatoren bilden, kann leicht dafür gesorgt werden, dass auch Deformationen, die diejenigen Flächen erfahren können, die den Messspalt begrenzen und an denen die Elektroden angeordnet sind, sich nicht auf das Messsignal auswirken. Da auf beiden Seiten des Messspaltes die Messelektrode, die Kompensationselektrode und die Anschlüsse dafür je auf einer gemeinsamen Fläche angeordnet sind, kann die Metallisierung, die diese Elektroden und Anschlüsse auf der Fläche bildet, sehr einfach auf der Fläche aufgetragen werden. Dies ist beispielsweise mit einem Siebdruckverfahren oder einem Aetzverfahren ohne Durchkontaktierungen möglich. Dies ist insbesondere dann vorteilhaft, wenn die Elektroden auf Keramikkörpem angeordnet sind, da diese nur sehr schwierig zu bearbeiten sind. Gemäss der Erfindung können somit beliebig viele Messspalte durch Aneinanderreihen von Platten, beispielsweise Keramikplatten, mit Elektroden an der Oberfläche in absolut symmetrischer Ausführung realisiert werden. Parasitärkapazitäten sind in diesem Falle gering, was hohe Messfrequenzen erlaubt und geringes Rauschen ergibt.

Im folgenden wird die Erfindung anhand eines Beispiels und mit Bezug auf die beiliegenden Figuren näher erläutert, wobei
Figur 1 schematisch die erfindungsgemässe Vorrichtung,
Figur 2 eine mögliche Schaltungsanordnung dazu,
Figur 3a bis 3c eine erste Anordnung von Kondensatorelektroden,
Figur 4a bis 4c eine zweite Anordnung von Kondensatorelektroden,
Figur 5a bis 5c eine dritte Anordnung von Kondensatorelektroden,
Figur 6 ein ungestörtes Signal,
Figur 7 ein gestörtes Signal,
Figur 8 eine Anordnung mit mehreren Messspalten,
Figur 9a bis 9c eine vierte Anordnung von Kondensatorelektroden,
Figur 10a bis 10c eine fünfte Anordnung von Kondensatorelektroden,
Figur 11a bis 11c eine sechste Anordnung von Kondensatorelektroden,
Figur 12 eine perspektivische Darstellung der Elektroden der sechsten Anordnung,
Figur 13 einen Teil der Vorrichtung,
Figur 14 eine perspektivische Ansicht der Rückseite der Vorrichtung und
Figur 15a, 15b eine weitere Anordnung von Elementen zeigt.

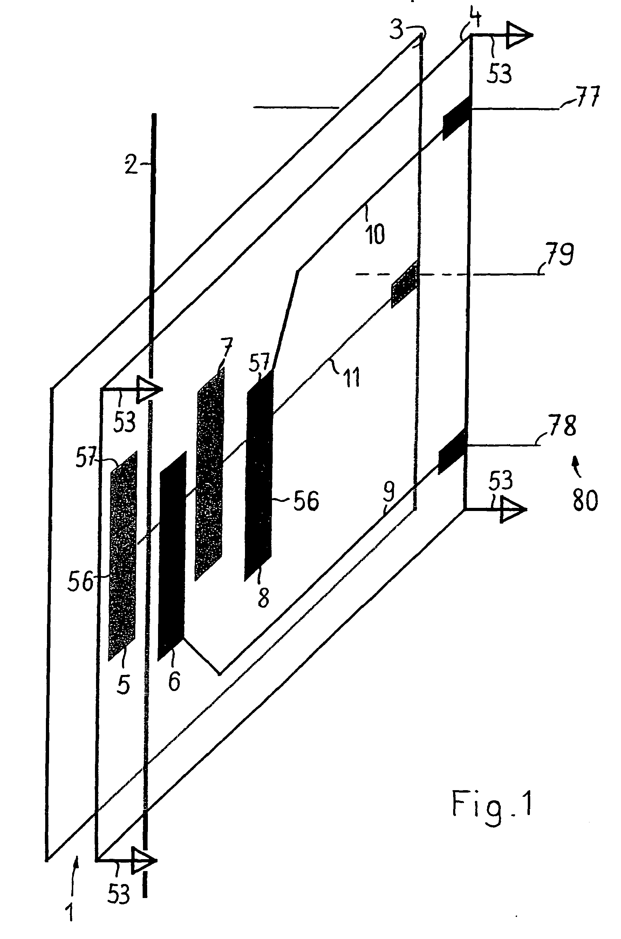

Figur 1 zeigt eine schematische Darstellung eines Messspaltes 1 für ein textiles Produkt 2 wie beispielsweise ein Band, ein Vorgarn oder ein Garn. Der Messspalt 1 ist im wesentlichen durch zwei Flächen 3 und 4 begrenzt. Elektroden 5, 6, 7 und 8 sind in diese Flächen 3 und 4 eingelassen, wobei es unerheblich ist, ob diese Elektroden 5 bis 8 aus den Flächen 3, 4 leicht vor- oder zurückstehen. Diese Elektroden 5, 6, 7, 8 sind über je eine eigene Leitung 9, 10 oder über eine gemeinsame Leitung 11 mit weiteren, hier nicht ersichtlichen Elementen einer Schaltung verbunden. Die Elektroden 5 und 6 sind so einandergegenüberliegend angeordnet, dass sie zusammen einen Messkondensator bilden. Die Elektroden 7 und 8 sind so zueinander angeordnet, dass sie einen Kompensationskondensator bilden. Das zu prüfende textile Produkt ist in an sich bekannter und deshalb hier nicht näher dargestellter Art so geführt, dass es zwischen den Elektroden 5 und 6 des Messkondensators liegt und eventuell in seiner Längsrichtung bewegt wird. Dabei werden weder die Elektroden 7, 8 des Kompensationskondensators, noch die Leitungen 9, 10 vom Produkt abgedeckt oder beschattet. Die Elektroden 5, 6, 7, 8 der Kondensatoren weisen je eine Längsseite 56 und eine Schmalseite 57 auf. Die Längsseiten der Elektroden 5, 6 des Messkondensators sind in einer beispielsweisen Ausführungsform mindestens annähernd parallel zur Längsrichtung des bewegten textilen Produktes 2. Geht man beispielsweise davon aus, dass mehrere Messspalte nebeneinander angeordnet werden sollen, so lassen sich Zuführungen 77, 78, 79 gleich für mehrere Messkondensatoren und Kompensationskondensatoren in einer Querebene 80 anordnen, wie sie durch die Zuführungen 77 - 79 aufgespannt wird.

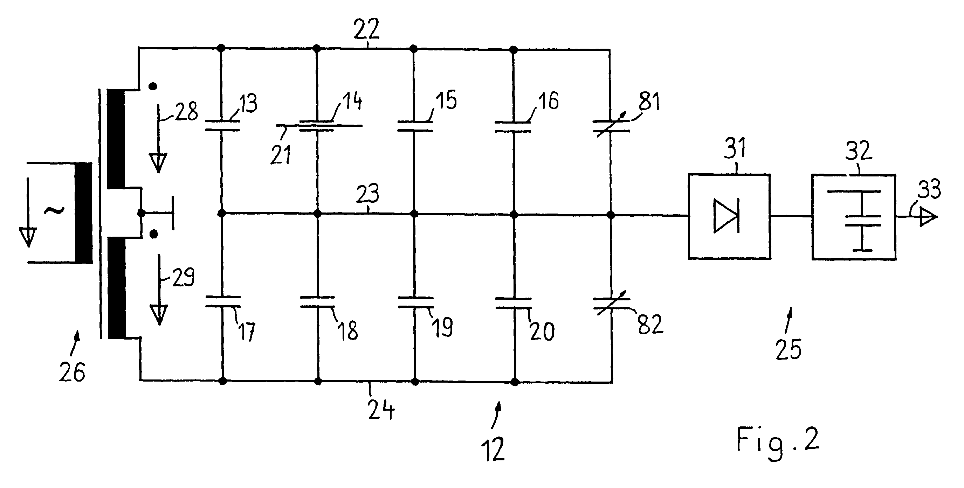

Figur 2 zeigt eine mögliche Schaltung 12 zur Auswertung von Signalen im Zusammenhang mit Messkondensatoren und Kompensationskondensatoren wie sie bereits aus der Figur 1 bekannt sind. Man erkennt darin beispielsweise vier Messkondensatoren 13, 14, 15, 16 und vier Kompensationskondensatoren 17, 18, 19, 20, sowie zwei Abgleich-Kondensatoren 81, 82. Dabei ist jeweils nur je ein Messkondensator mit einem textilen Produkt 21 versehen, wie dies hier für den Messkondensator 14 gezeigt ist. Zusammenwirkende Kondensatorpaare 13-17, 14-18, 15-19, und 16-20 sind hier parallel geschaltet und an Leitungen 22, 23 und 24 angeschlossen, die diese Kondensatorpaare einerseits mit einer Auswerteschaltung 25 und andererseits mit einer Signalquelle 26 verbinden. Als Signalquelle 26 ist hier beispielsweise ein Sinusoszillator, welcher zwei gegenphasige Signale oder Wechselspannungen 28 und 29 abgibt, vorgesehen. Die Auswerteschaltung 25, die über die Leitung 23 an die Kondensatoren 13 bis 20 angeschlossen ist, besteht hier beispielsweise aus einem Gleichrichter 31 und einem Filter 32, die in Serie geschaltet sind. Am Ausgang 33 ist damit ein Signal verfügbar, das Eigenschaften des gemessenen Produktes wiedergibt. Die Signalquelle 26 bildet zusammen mit je zwei Kondensatoren 13, 17 oder 14, 18 usw. eine Brückenschaltung. Die Brückenschaltung kann auch so aufgefasst werden, dass die Kondensatoren 13 bis 16 und die Kondensatoren 17 bis 20 zusammen jeweils einen Zweig der Brückenschaltung bilden.

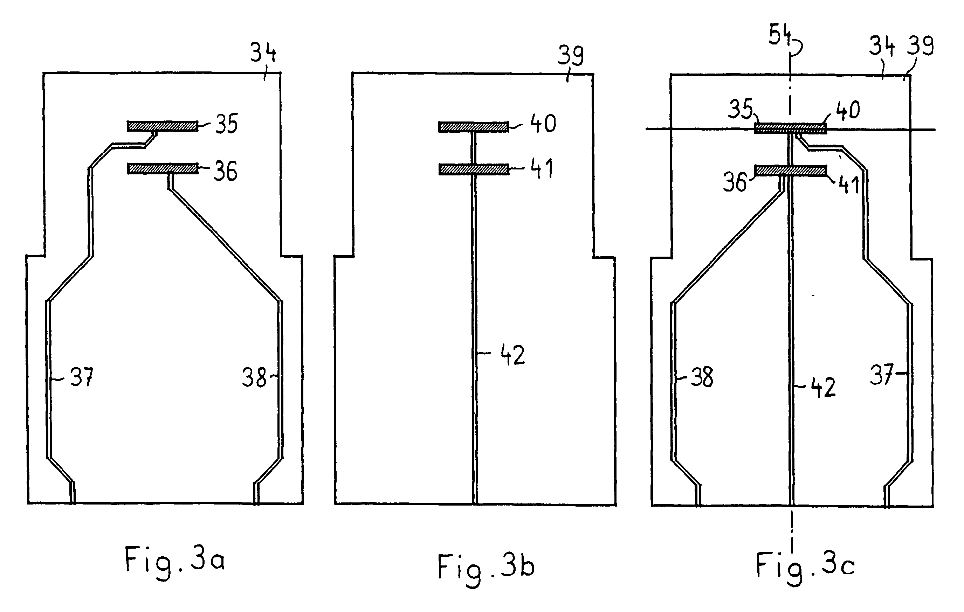

Figur 3a zeigt in schematischer Darstellung eine Aufsicht auf eine Fläche 34 eines Messspaltes mit einer Elektrode 35, die zum Messkondensator gehört und einer Elektrode 36, die zum Kompensationskondensator gehört. Ebenfalls ersichtlich sind Leitungen 37 und 38 die zu diesen Elektroden führen.

Figur 3b zeigt in entsprechender Weise eine Fläche 39, die zusammen mit der obengenannten Fläche 34 einen Messspalt bildet. Hier sind Elektroden 40 des Messkondensators und 41 des Kompensationskondensators über eine gemeinsame Leitung 42 verbunden und an die übrigen Schaltungsteile angeschlossen.

Figur 3c zeigt die beiden nun bekannten Flächen 34, 39 die einander gegenübergestellt sind, so dass sie einen Messspalt bilden. Daraus erkennt man auch die Führung der Leitungen 37, 38 und 42, die so gewählt ist, dass diese weit auseinander liegen, damit die gegenseitige Beeinflussung gering bleibt.

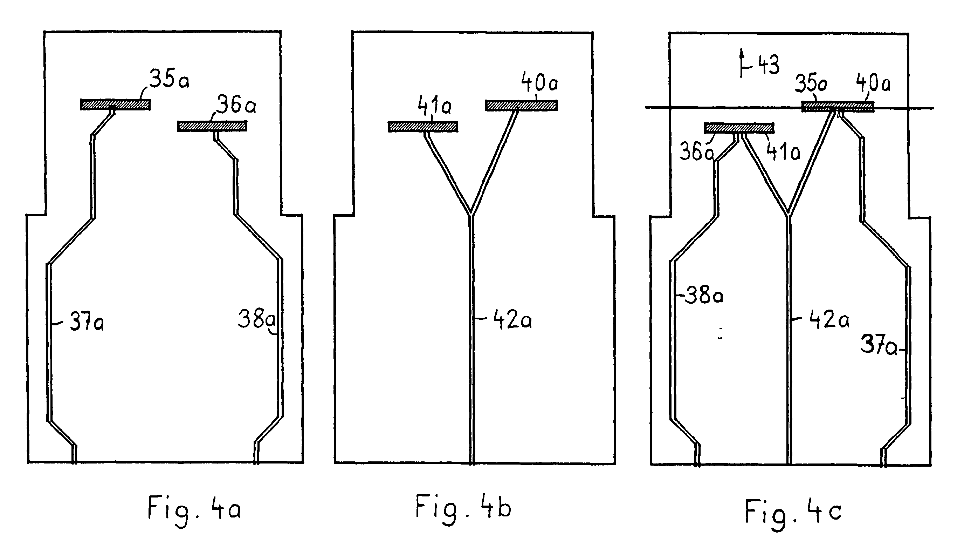

Die Figuren 4a, 4b und 4c zeigen eine weitere Anordnung von Elektroden 35a, 36a, 40a, 41a und Leitungen 37a, 38a, 42a. Die beiden Elektrodenpaare 35a, 40a und 36a, 41a sind dabei so angeordnet, dass sie in Richtung eines Pfeiles 43 gesehen möglichst nahe zueinander liegen, einander aber nicht überdecken.

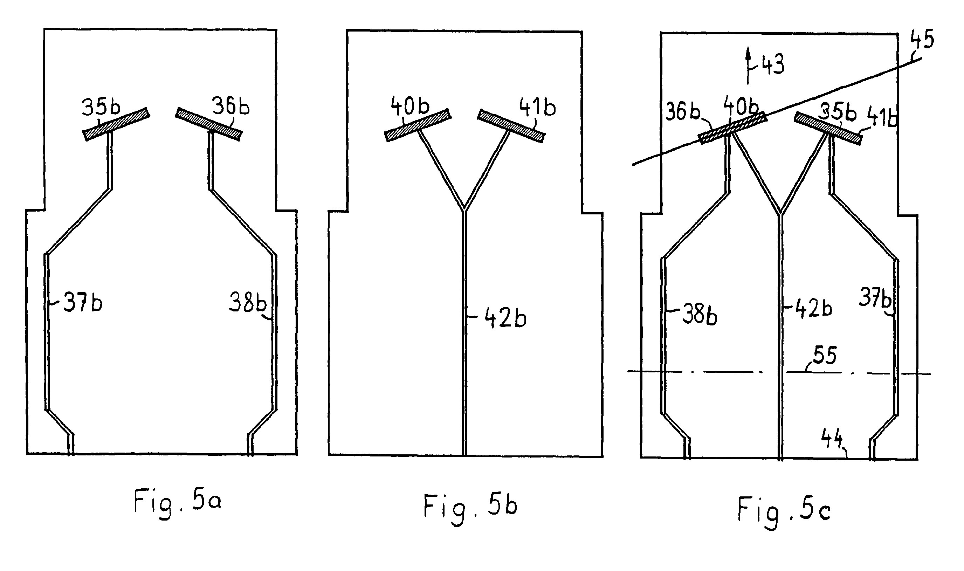

Die Figuren 5a, 5b und 5c zeigen eine weitere Anordnung von Elektroden 35b, 36b, 40b, 41b und Leitungen 37b, 38b, 42b. Die beiden Elektrodenpaare 36b, 40b und 35b, 41b sind dabei so angeordnet, dass sie in Richtung eines Pfeiles 43 gesehen möglichst auf gleicher Höhe oder in gleichem Abstand von einer Referenzlinie 44 zueinander liegen, einander aber nicht überdecken. Damit ist auch dafür gesorgt, dass das Produkt 45 nicht zwischen die Elektroden 35b, 41b des Kompensationskondensators zu liegen kommt. Dafür ist auch bei den vorgehend beschriebenen Ausführungen selbstverständlich gesorgt.

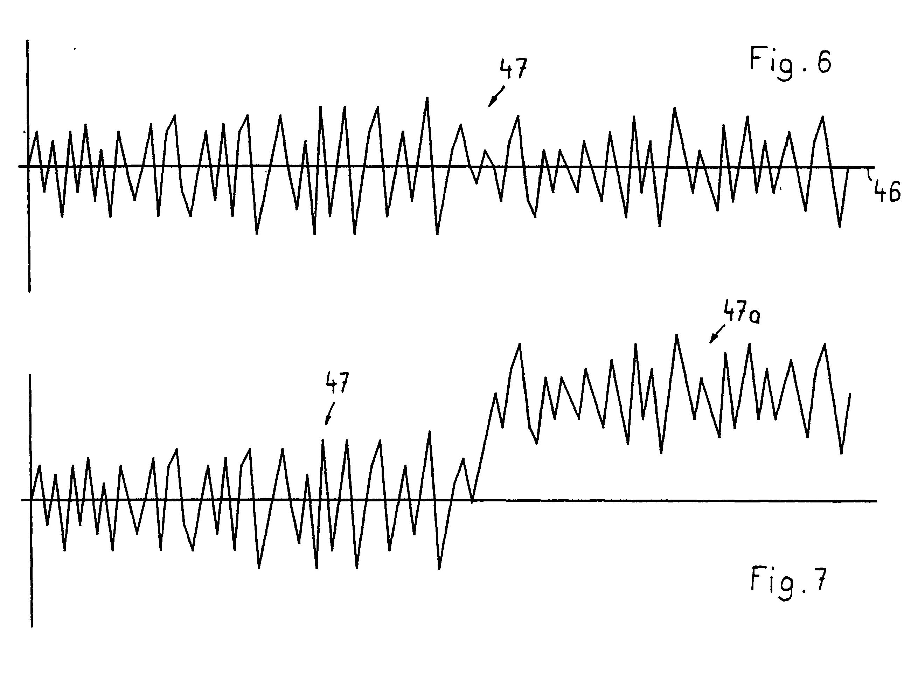

Figur 6 zeigt über einer Zeitachse 46 aufgetragen, ein Signal 47, dessen Auslenkungen Eigenschaften des zu messenden Produktes anzeigen. Eine solche Eigenschaft ist beispielsweise die Masse oder der Querschnitt des Produktes. Das Signal 47 gibt hier insbesondere Abweichungen solcher Eigenschaften von einem Durchschnittswert als Funktion der Zeit an.

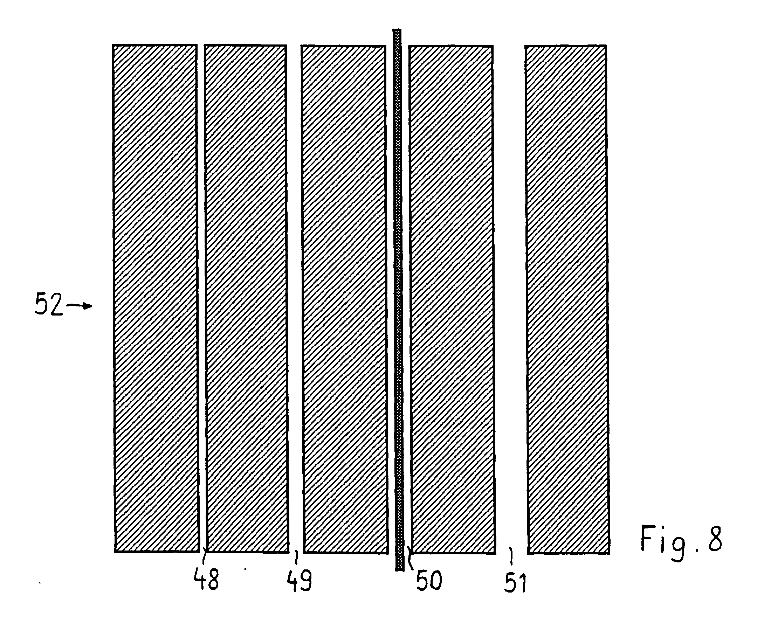

Figur 7 zeigt ein Signal 47 wie es bereits aus der Figur 6 bekannt ist, das aber durch äussere Störeinflüsse in einem Bereiche 47a ausgelenkt ist.

Figur 8 zeigt eine Anordnung von mehreren Messpalten 48, 49, 50 und 51 verschiedener Breite, die in einem einzigen Element 52 gemeinsam angeordnet sind. Jeder Messspalt eignet sich zum Messen von Produkten mit anderen Massen. Dicke Produkte sind im Messspalt 51, dünne Produkte sind im Messspalt 48 zu messen.

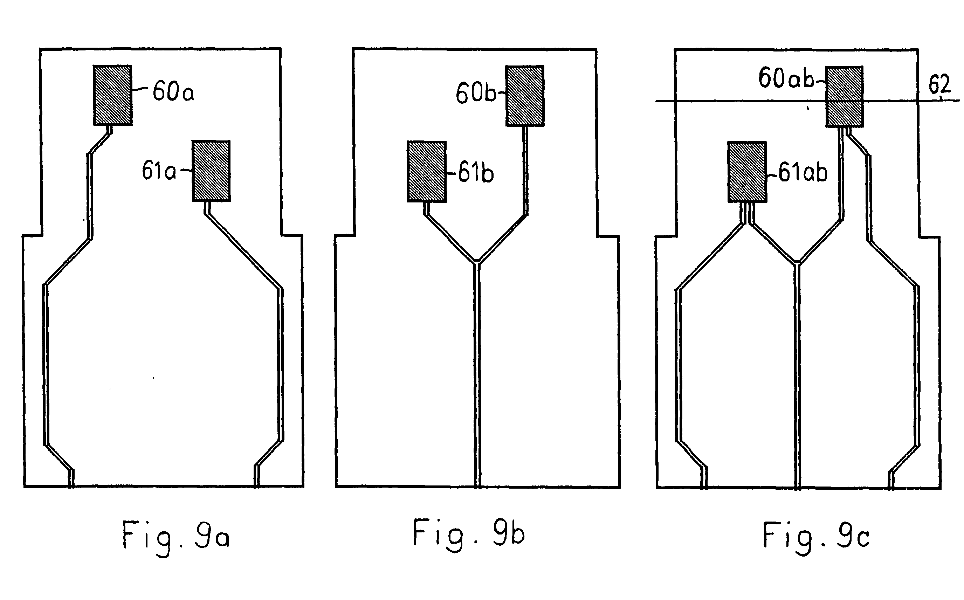

Figuren 9a, 9b, 9c zeigen in nun bekannter Weise weitere schematische Darstellungen von Elektroden 60a, 60b, bzw. 60ab in übereinandergelegter Stellung, die einen Messkondensator bilden, sowie von Elektroden 61a, 61b, bzw. 61ab, die einen Kompensationskondensator bilden. Die Elektroden haben hier eine Ausdehnung, die nicht mehr in Längsrichtung des Produktes 62, sondern hauptsächlich quer dazu verläuft. Damit kann das Produkt verschiedene Stellungen einnehmen und muss nicht mehr so genau geführt werden wie das bei den bisher gezeigten Ausführungen der Fall war. Oder, es können andere, insbesondere dikkere Produkte wie z.B. Vorgame oder Bänder gemessen werden.

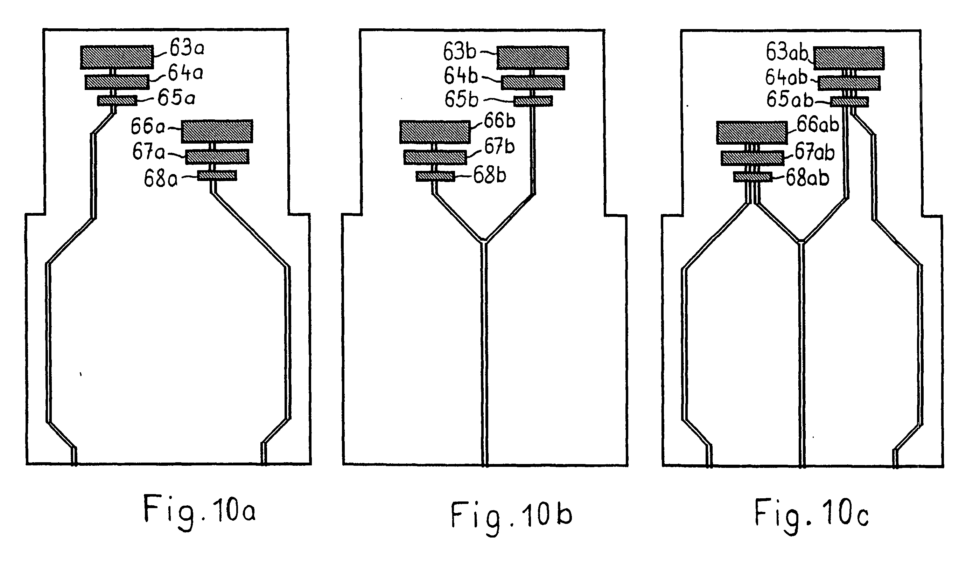

Eine weitere Ausführung ist in den Figuren 10a, 10b und 10c gezeigt, in der jeweils drei Elektroden 63a, 64a und 65a, sowie 63b, 64b, 64c für drei Messkondensatoren nebeneinander angeordnet sind. Entsprechend sind auch Elektroden 66a, 67a, 68a sowie 66b, 67b, 68b für Kompensationskondensatoren nebeneinander angeordnet. Dabei eignen sich die Elektroden 63 hauptsächlich für dickere Produkte während die Elektroden 65 für dünne Produkte geeignet sind. So sind drei spezialisierte Messkondensatoren und drei entsprechende Kompensationskondensatoren in einem Messpalt angeordnet. Zusätzlich ist es auch denkbar, den Messpalt entsprechend in seiner Breite abzustufen, so dass er im Bereiche der Elektroden 63 weiter geöffnet ist als im Bereiche der Elektroden 65.

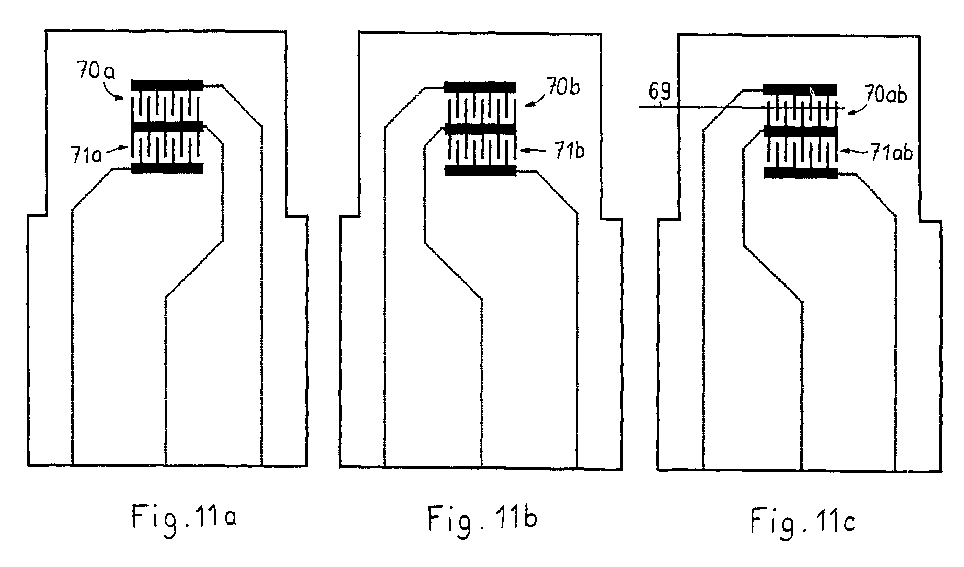

Eine weitere Möglichkeit wird in den Figuren 11a, 11b und 11c gezeigt. Hier sind Elektrodenfelder 70a und 70b für einen Messkondensator für ein Produkt 69 und Elektrodenfelder 71a und 71b für einen Kompensationskondensator vorgesehen. Die elektrischen Felder verlaufen hier nicht mehr quer zum Produkt wie in den bisher gezeigten Anordnungen, sondern längs dazu.

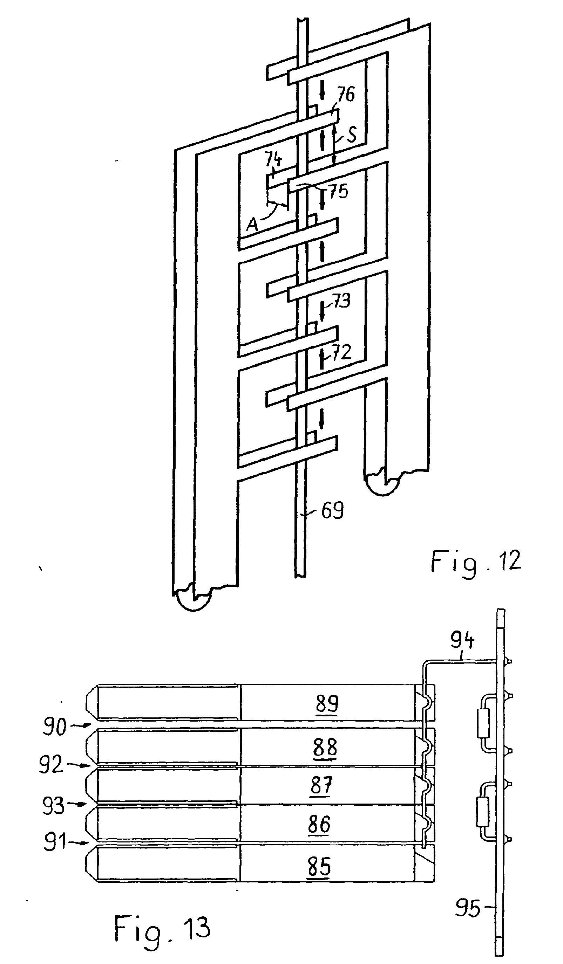

Dies geht insbesondere aus der Figur 12 hervor, wo eine perspektivische Darstellung eines Kondensators mit solchen Elektrodenfeldem dargestellt ist. Man erkennt hier das Produkt 69 und parallel dazu verlaufende und durch Pfeile 72 und 73 angedeutete Feldlinien. Um ein möglichst homogenes Feld zu erhalten, wird das Leiterbild nicht nur auf eine Platte aufgebracht, sondern es werden wie bei den Querfeldsensoren zwei Platten verwendet, wobei sich auf beiden Platten das gleiche Leiterbild gegenübersteht. Die Sensitivität wird dabei nicht wie beim Querfeld-Sensor durch den Abstand A zwischen den Stegen 74 und 75 sondern durch den Stegabstand S zwischen Stegen 75 und 76 des gleichen Leiterbildes oder Elektrodenfeldes. Bei diesem Sensor lässt sich die Drift mit dem Kompensationsfeld besonders stark verbessem, da die Kapazitäten sehr klein sind und damit eine sehr grosse Störempfindlichkeit besteht.

Fig. 13 zeigt eine Anordnung von fünf Trägerplatten 85, 86, 87, 88 und 89, die vier Messspalte 90, 91, 92 und 93 unterschiedlicher Spaltbreite bilden. Vorzugsweise sind die Messspalte 90 und 91 mit der grösseren Spaltbreite aussen und die Messspalte 92, 93 mit der kleineren Spaltbreite innen angeordnet. Die Elektroden in den Messspalten 90 bis 93 sind über Leitungen, von denen hier nur eine Leitung 94 sichtbar ist, mit einer Leiterplatte 95 verbunden, auf der beispielsweise die Elemente der genannten Brükenschaltungen oder der in Fig. 2 gezeigten Schaltung angeordnet sind.

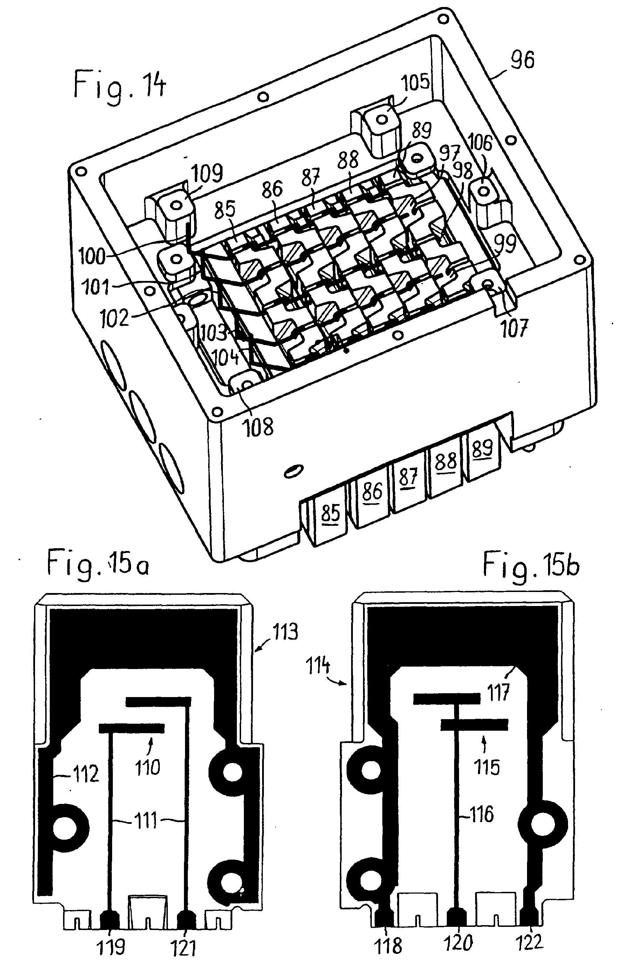

Fig. 14 zeigt die Anordnung der Trägerplatten 85 bis 89 gemäss Fig. 13 in einem Gehäuse 96 von hinten. Jede dieser Trägerplatten 85 bis 89 weist an ihrer rückwärtigen Stirnfäche mehrere Ausnehmungen 97, 98 und 99 auf, die einen Teil einer Seitenfläche freilegen, auf der die Elektroden 5, 6, 7, 8 und Leiter 9, 10, 11 gemäss Fig. 1 angeordnet sind. Dies schafft einen Zugang für ein Lötgerät, mit dem Zuführungen 100, 101, 102, 103, 104 angeschlossen wer den können. Die genannten Zuführungen 101 - 103 entsprechen beispielsweise den Leitungen 22, 23, 24 gemäss Fig. 2. Zuführungen 100 und 104 sind für Abschirmungen vorgesehen. Die aus der Fig. 13 bekannte Leiterplatte 95 kann im Gehäuse 96 auf Abstützungen 105 bis 109 festgemacht werden.

Fig. 15a zeigt eine Ansicht einer Trägerplatte 113, auf der neben Elektroden 110 und Leitern 111 auch eine Abschirmung 112 angeordnet ist. Fig. 15b zeigt eine Trägerplatte 114 mit entsprechenden Elektroden 115, die mit den Elektroden 110 Kondensatoren bilden, einer Leitung 116 und einer Abschirmung 117. Anschlüsse 118 bis 122 dienen zur Verbindung mit Leitungen 100 bis 104 gemäss Fig. 14. Die Abschirmung schirmt einerseits das Messfeld in den Kondensatoren gegen Störeinflüsse von aussen ab. Andererseits sorgt sie auch dafür, dass das Messfeld Störungen nicht nach aussen trägt.

Die Arbeitsweise der erfindungsgemässen Vorrichtung ist beispielsweise wie folgt:
Mit einem Übertrager oder einem Oszillator werden zwei gleich grosse, aber gegenphasige Wechselspannungen 28 und 29 (Fig. 2) erzeugt. Bei abgeglichener Brücke und ohne Prüfgut 21 haben die Messkondensatoren 13 bis 16 gleich grosse Werte wie die Kompensationskondensatoren 17 bis 20. An der Leitung 23 liegt damit keine Spannung an. Wird das Produkt 21 in einen Messspalt eingeführt, ändert sich das Gleichgewicht zwischen den Messkondensatoren und den Kompensationskondensatoren und in der Leitung 23 wird eine Wechselspannung erzeugt, deren Amplitude proportional zur Masse des Produktes zwischen den Messelektroden ist. Nach Gleichrichtung und Filterung im Gleichrichter 31 und Filter 32 erhält man ein Signal 33, dessen zeitlicher Verlauf der Envelope des Wechselstromsignales in der Leitung 23 entspricht und das damit dem Massenverlauf des Prüfgutes entspricht, wenn dieses durch den Messspalt hindurch bewegt wird. Dieses Signal ist aus der Fig. 6 bekannt.

Durch die erfindungsgemässe Anordnung können aber Störeinflüsse neutralisiert werden. Ein solcher Störeinfluss ist beispielsweise die mechanische Verschiebung der Flächen 3 und 4 die den Messspalt 1 bilden oder unterschiedliche Luft- oder Feuchtigkeitsverhältnisse. Weitet sich der Messspalt 1 infolge mechanischer oder thermischer Belastung in Richtung von Pfeilen 53 (Fig. 1) auf, so nimmt die Dichte des elektrischen Feldes zwischen den Elektroden 5 und 6 ab und ein Produkt 2 ergibt ein kleineres Signal als bei der ursprünglichen Stellung der Elektroden 5 und 6. Da aber die Elektroden 5 und 6 an eine Schaltung gemäss Fig. 2 angeschlossen sind, ist nicht der Zustand des Feldes zwischen den Elektroden 5 und 6 allein massgebend sondern es ist der Zustand zwischen den Elektroden 5 und 6 gemessen am Zustand zwischen den Elektroden 7 und 8 massgebend und dieser relative Zustand ändert sich nicht, wenn die Elektroden 7 und 8 in gleichem Masse wie die Elektroden 5 und 6 voneinander entfernt werden. Der Nullpunkt verschiebt sich dadurch nicht.

Es ist ebenfalls denkbar, dass sich die beiden Flächen 3 und 4 durch die genannten Störeinflüsse nicht parallel verschieben, sondern durch den Störeinfluss nicht mehr eine parallele Lage einnehmen. In diesem Falle kann der absolute Betrag des Signales 47 zwar eine Änderung erfahren, das Signal bleibt aber trotzdem einigermassen zentriert. Allerdings können besondere Anordnungen der Elektroden solche Fälle besonders berücksichtigen. Beispielsweise ist die Ausführung gemäss Fig. 3 besonders geeignet, eine Verdrehung der einen Fläche gegenüber der anderen Fläche um eine Achse 54 zu kompensieren. Dagegen ist die Ausführung gemäss Fig. 5 besonders geeignet, eine Verdrehung der einen Fläche um eine Achse 55 zu kompensieren.

Besonders vorteilhaft ist die erfindungsgemässe Lösung bei einer Anordnung von mehreren Messspalten 48 bis 51 nebeneinander, wie dies die Figur 8 zeigt. Die Elektroden dieser Messspalte 48 bis 51 können alle in einer gemeinsamen Schaltung angeordnet und miteinander verbunden sein. Wird beispielsweise der Messspalt 50 aus irgend einem Grunde verkleinert, so hat dies wahrscheinlich zur Folge, dass einer der benachbarten Messspalte 49 oder 51 eine entsprechende Vergrösserung erfährt. In der Schaltung gemäss Fig, 2 bedeutet dies beispielsweise, dass die Spalte in den Kondensatoren 14 und 18 verkleinert, die Spalte in den Kondensatoren 13 und 17 oder 15 und 19 entsprechend vergrössert werden. Daraus lässt sich leicht erkennen, dass sich damit der Nullpunkt nicht verschiebt. Durch die Anordnung der Leitungen in einer Ebene mit den Elektroden und durch die nur einseitig, also zum Prüfgut unsymmetrisch angeordneten Kompensationskondensatoren, lässt sich ein sehr kompakter Messkopf bauen. Vorzugsweise werden die Oberflächen der Platten, die einen Messspalt bilden, durch eine Beschichtung, z. B. eine Glasschicht abgedeckt und geschützt, so dass die Metallisierungen, die die Elektroden und Zuleitungen bilden entsprechend dünnschichtig ausgebildet sein können.

Das gleiche Verhalten zeigt sich auch bei anderen Störeinflüssen wie z. B. Temperaturdifferenzen zwischen den einzelnen Messspalten, Feuchtigkeitsdifferenzen, anderen Luftströmungen oder Alterungen des Materials.

Es ist mit der erfindungsgemässen Lösung auch denkbar einen einzigen Messspalt vorzusehen dessen Flächen 3 und 4 durch einen beliebigen Mechanismus in Richtung der Pfeile 53 (Fig. 1) so zueinander einstellbar sind, dass Produkte verschiedenster Dicke darin gemessen werden können. Dabei erfolgt die richtige Einstellung eines Kompensationskondensators automatisch.

Ebenso können andere Auswerteschaltungen vorgesehen werden. Eine solche Schaltung ist beispielsweise aus der Patentanmeldung DE 36 21 324 bekannt. Immer wird dabei die Messkapazität mit der Kompensationskapazität verglichen, sei es direkt oder durch Umwandlung der Kapazität in eine andere physikalische Grösse wie z.B. eine Spannung, Frequenz, oder einen Strom.

## Patentansprüche

1. Vorrichtung zum Messen von Eigenschaften eines textilen Produktes (2) in einem Messspalt (1), in dem das textile Produkt eingeführt ist, wobei der Messspalt Elektroden (5, 6) eines Messkondensators, Elektroden (7, 8) eines Kompensationskondensators und Leitungen (9, 10, 11) zu beiden Kondensatoren aufweist, **dadurch gekennzeichnet, dass** die Leitungen in einer Ebene mit den Elektroden angeordnet sind.

2. Vorrichtung nach Anspruch 1, **dadurch gekennzeichnet, dass** der Messkondensator und der Kompensationskondensator in Serie an eine Signalquelle (26) angeschlossen sind.

3. Vorrichtung nach Anspruch 1, **dadurch gekennzeichnet, dass** der Messkondensator und der Kompensationskondensator je gleich viele Elektroden aufweisen.

4. Vorrichtung nach Anspruch 1, **dadurch gekennzeichnet, dass** Elektroden des Messkondensators und des Kompensationskondensators zueinander geneigt angeordnet sind.

5. Vorrichtung nach Anspruch 1, **dadurch gekennzeichnet, dass** einander zugeordnete Elektroden, die einen Kondensator bilden, ein quer über den Messspalt gerichtetes Feld erzeugen.

6. Vorrichtung nach Anspruch 1, **dadurch gekennzeichnet, dass** einander zugeordnete Elektroden, die einen Kondensator bilden, ein zum Produkt längs gerichtetes Feld zeugen.

7. Vorrichtung nach Anspruch 1, **dadurch gekennzeichnet, dass** die Elektroden und Leitungen auf einer Trägerplatte (85 - 89) aufgebracht sind, die an einer Stirnfläche Ausnehmungen (97, 98, 99) zur Verbindung der Leitungen mit Zuführungen (101 - 103) aufweisen.

## Claims

1. Device for measuring properties of a textile product (2) in a measuring gap (1) in which the textile product is inserted, whereas the measuring gap has electrodes (5, 6) of a measuring capacitor, electrodes (7, 8) of a compensation capacitor and conductors (9, 10, 11), **characterized in that** the conductors are arranged in a plane with the electrodes.

2. Device according to claim 1, **characterized in that** the measuring capacitor and the compensation capacitor are connected in series to a signal source (26).

3. Device according to claim 1, **characterized in that** the measuring capacitor and the compensation capacitor each have the same number of electrodes.

4. Device according to claim 1, **characterized in that** electrodes of the measuring capacitor and of the compensation capacitor are arranged inclined with respect to each other.

5. Device according to claim 1, **characterized in that** electrodes allocated to each other, which form a capacitor, generate a field directed crosswise over the measuring gap.

6. Device according to claim 1, **characterized in that** electrodes allocated to each other, which form a capacitor, generate a field directed lengthwise to the product.

7. Device according to claim 1, **characterized in that** the electrodes and conductors are accomodated on a carrier board (85 - 89) which on a front face has recesses (97, 98, 99) to connect the conductors to supply conductors (101 - 103).

## Revendications

1. Dispositif pour la mesure de caractéristiques d'un produit textile (2) dans une fente de mesure (1), dans laquelle le produit textile est inséré, où la fente de mesure présente des électrodes (5, 6) d'un condensateur de mesure, des électrodes (7, 8) d'un condensateur de compensation et des lignes (9, 10, 11) vers les deux condensateurs, **caractérisé en ce que** les lignes sont disposées dans un plan avec les électrodes.

2. Dispositif selon la revendication 1, **caractérisé en ce que** le condensateur de mesure et le condensateur de compensation sont connectés en série à une source de signaux (26).

3. Dispositif selon la revendication 1, **caractérisé en ce que** le condensateur de mesure et le condensateur de compensation présentent le même nombre d'électrodes.

4. Dispositif selon la revendication 1, **caractérisé en ce que** les électrodes du condensateur de mesure et du condensateur de compensation sont disposées selon une inclinaison les unes aux autres.

5. Dispositif selon la revendication 1, **caractérisé en ce que** les électrodes associées l'une à l'autre, qui forment un condensateur, produisent un champ dirigé transversalement sur la fente de mesure.

6. Dispositif selon la revendication 1, **caractérisé en ce que** des électrodes associées l'une à l'autre, qui forment un condensateur, produisent un champ dirigé longitudinalement au produit.

7. Dispositif selon la revendication 1, **caractérisé en ce que** les électrodes et les lignes sont appliquées sur une plaque de support (85-89) qui présente à une face frontale des évidements (97,98,99) pour relier les lignes à des lignes d'amenée (101-103).
